# EUROPÄISCHE PATENTANMELDUNG

(11) **EP 1 512 460 A1**
(43) Veröffentlichungstag der Anmeldung: **09.03.2005**
(21) Anmeldenummer: 03102712.1
(22) Anmeldetag: 05.09.2003
(51) Int. Cl.: B01J 31/00, C07D 213/20, C07D 233/54

(54) **Herstellung und Verwendung von ionischen Flüssigkeiten mit Thiocyanat als Anion**

(71) Anmelder: Solvent Innovation GmbH, 50829 Köln (DE)
(72) Erfinder: Uerdingen, Marc Dr., 53797, Lohmar (DE); Hilgers, Claus Dr., 50829, Köln (DE)
(74) Vertreter: Weber, Thomas, Dr.

(57) **Zusammenfassung**

Die Erfindung betrifft ionische Flüssigkeiten der allgemeinen Formel [X⁺][SCN⁻], ein verbessertes Verfahren zu deren Synthese, sowie deren Verwendung in der Elektrochemie, zur Stofftrennung und in chemischen Reaktionen.

## Beschreibung

Die Erfindung betrifft ionische Flüssigkeiten der allgemeinen Formel [X⁺][SCN⁻], ein Verfahren zu deren Synthese, sowie deren Verwendung in der Elektrochemie, bei der Stofftrennung und in chemischen Reaktionen.

Unter ionischen Flüssigkeiten versteht man im Allgemeinen Salze oder Gemische aus Salzen mit Schmelzpunkten bei Normaldruck (101,325 kPa) unterhalb 100°C (P. Wasserscheid, W. Keim, *Angew. Chem.* 2001, *112*, 3926). Literaturbekannte Salze dieser Art bestehen aus Anionen wie z. B. Halogenostannaten, Halogenoaluminaten, Hexafluorophosphaten, Tetrafluoroboraten, Alkylsulfaten, Alkyl- oder Arylsulfonaten kombiniert mit substituierten Ammonium-, Phosphonium, Pyridinium- oder Imidazolium-Kationen. Zahlreiche Veröffentlichungen beschreiben bereits die Verwendung ionischer Flüssigkeiten als Lösungsmittel für chemische Reaktionen (T. Welton, Chem. Rev. 1999, *99,* 2071, P. Wasserscheid, W. Keim, *Angew. Chem.,* 2000, *112*, 3926). Weitere wichtige Einsatzfelder ionischer Flüssigkeiten liegen in ihrer Verwendung als Extraktionsmittel zur Stofftrennung (J. G. Huddleston, H. D. Willauer, R. P. Swatloski, A. E. Visser, R. D. Rogers, *Chem. Commun.* 1998, 1765-1766; b) A. E. Visser, R. P. Swatlowski, R. D. Rogers, *Green Chemistry* 2000, *2(1),* 1-4) sowie in ihrer Verwendung als Elektrolyt z. B. in Sensoren, Batterien, Farbstoffsolarzellen, Kondensatoren und zur elektrolytischen Abscheidung von Metallen (P. Bonhöte, A.-P. Dias, N. Papageorgiou, K. Kalyanasundaram, M. Grätzel, Inorg. Chem. 1996, 35, 1168-1178) - wobei diese Beispiele keinen Anspruch auf Vollständigkeit erheben.

Golding *et al* offenbaren in einer im Jahre 2002 erschienenen Publikation (J. M. Pringle, J. Golding, C. M. Forsyth, G. B. Deacon, M. Forsyth, D. R. MacFarlane, 2002, *12*, 3475-3480) eine Reihe von Pyrrollidinium-Salzen mit Thiocyanat-lon, die Verbindungen 1-Ethyl-3-methylimidazolium-Thiocyanat und N-Hexyl-N,N,N-tributylammonium-Thiocyanat und deren thermische (DSC und TGA-Analyse) sowie die elektrochemische Eigenschaften (Cyclovoltagramme). Ferner sind Details zur Kristallstruktur spezieller Pyrrollidinium-Thiocayante und Betrachtungen zu deren Festkörpereigenschaften beschrieben.

Alle beschriebenen Thiocyanate wurden im Maßstab < 10g durch Umsetzung der entsprechenden Amine mit einem Alkyliodid, gefolgt von einer Fällungsreaktion mit Silberthiocyanat erhalten.

Die von Golding *et al* offenbarte Herstellungsroute ist jedoch gänzlich ungeeignet, um die beschriebenen Substanzen in technischen Mengen herzustellen. Allein der Preis des Silbersalzes verbietet eine Maßstabvergrößerung. Dazu kommen die Entsorgungskosten für das als Nebenprodukt gebildete Silberiodid.

Schwerer wiegt, insbesondere im Hinblick auf die potentielle technische Verwendung dieser Salze in Anwendungen der Elektrochemie, Stofftrennung und chemischen Reaktion, jedoch die mangelhafte Qualität, in der die beschriebenen Thiocyanatsalze nach der von Golding *et al* offenbarten Synthese erhalten werden. Die Autoren selbst beschreiben diese Problematik explizit. Sie weisen darauf hin, dass die von ihnen erhaltenen und charakterisierten Substanzen noch bis zu 5000 ppm Ag⁺ und I⁻ Ionen als Verunreinigungen enthalten und warnen ausdrücklich vor den negativen Einflüssen der Verunreinigungen auf die oxidative Stabilität der so hergestellten ionischen Flüssigkeiten. Vor allem die Verunreinigung durch das Iodid-Ion ist problematisch, da dieses bekanntermaßen leicht zu Iod oxidiert wird und in dieser Form die Lichtstabilität der Salze dramatisch verringert. Die Autoren stellen explizit fest, dass die Anwesenheit von Iod oder Brom sehr wahrscheinlich zu einer elektrochemischen Oxidation von Teilen des Thiocyanat-Ions unter Bildung einer polymeren Spezies führt. Mit der beschriebenen Polymerisation geht die oxidative Zersetzung der ionischen Flüssigkeit einher.

Im Hinblick auf die im Zusammenhang mit ihrer offenbarten Herstellungsmethode für die beschriebenen ionischen Flüssigkeiten bestehenden Probleme berichten Golding *et al* auch über ihren fehlgeschlagenen Versuch, die beschriebenen Substanzen mittels Ionenaustauscherharzen herzustellen. Es gelingt ihnen auf diese Weise jedoch nicht, qualitativ hochwertige Produkte zu erhalten. Die Autoren stellen fest, dass die Zugabe einer wässrigen Lösung des Iodidsalzes zu einem mit KSCN beladenen Ionentauscher kein sauberes Produkt liefert. Daher wurden alle von Golding *et al* offenbarten ionischen Flüssigkeiten über die Fällung von Silberiodid hergestellt.

Der vorliegenden Erfindung liegt daher die Aufgabe zugrunde, die zuvor beschriebenen Probleme bei der Herstellung von ionischen Flüssigkeiten mit Thiocyanato-Anionen zu überwinden.

Ein erster Gegenstand der vorliegenden Erfindung betrifft daher ein Verfahren zur Herstellung von ionischen Flüssigkeiten der allgemeinen Formel [X⁺][SCN⁻], wobei X⁺ ein hydrophiles oder hydrophobes Kation ist, wobei das Verfahren die folgenden Schritte umfasst:
(a) die Herstellung eines Salzes [X⁺][A⁻];
(b) das Umsetzen des in Schritt (a) erhaltenen Salzes [X⁺][A⁻] mit einem Thiocyanat-Salz [Y⁺][SCN⁻] in einem Zweiphasensystem aus Wasser und einem nicht mit Wasser mischbaren organischen Lösungsmittel;
(c) die Isolierung der ionischen Flüssigkeit [X⁺][SCN⁻] aus einer der beiden Phasen, wobei
   - im Falle eines hydrophilen Kations [X⁺] in Schritt (a) ein Salz [X⁺][A⁻] mit einem hydrophoben Anion [A⁻] hergestellt wird, welches in Schritt (b) mit einem Thiocyanat-Salz [Y⁺][SCN⁻] mit hydrophobem Kation [Y⁺] umgesetzt wird, und
   - im Falle eines hydrophoben Kations [X⁺] in Schritt (a) ein Salz [X⁺][A⁻] mit einem hydrophilen Anion [A⁻] hergestellt wird, welches in Schritt (b) mit einem Thiocyanat-Salz [Y⁺][SCN⁻] mit hydrophilen Kation [Y] umgesetzt wird.

Die Herstellung der Salze [X⁺][A⁻] mit dem gewünschten Kation [X⁺] kann nach einem herkömmlichen, im Stand der Technik bekannten und einleitend in Bezug genommenen Verfahren erfolgen.

Das erfindungsgemäße Herstellungsverfahren zur Herstellung von ionischen Flüssigkeiten mit hydrophilem Kation [X⁺] wird nachfolgend als "Variante 1", das Verfahren Herstellung ionischer Flüssigkeiten mit hydrophobem Kation [X⁺] wird nachfolgend als "Variante 2" bezeichnet.

Das erfindungsgemäße Verfahren liefert ionische Flüssigkeiten der Formel [X⁺][SCN⁻], welche im wesentlichen frei sind von Ag⁺- und I⁻-Ionen, worunter man jeweils Ag⁺- und I⁻-Konzentration von <5 ppm und vorzugsweise <3 ppm, im Idealfall 0 ppm versteht. Durch die Abwesenheit von Ag⁺- und I⁻-Ionen eröffnen sich völlig neue Verwendungsmöglichkeiten der erfindungsgemäß hergestellten ionischen Flüssigkeiten.

Das erfindungsgemäße Verfahren ermöglicht eine einfache und vorzugsweise vollständige Abtrennung der entstehenden ionischen Flüssigkeit [X⁺][SCN⁻], die sich in der einen der beiden Phasen anreichert vom Nebenprodukt [Y⁺][A⁻], welches sich in der jeweils anderen Phase anreichert. Im Falle der 1. Variante, reichert sich die hydrophile ionische Flüssigkeit [X⁺][SCN⁻] in der Wasserphase und das hydrophobe Salz [Y⁺][A⁻] in der organischen Phase an. Im andern Fall (2. Variante) löst sich die hydrophobe ionische Flüssigkeit [X⁺][SCN⁻] in der organischen Phase, während sich das hydrophile Salz [Y⁺][A⁻] in der Wasserphase anreichert.

Die Isolierung der ionischen Flüssigkeit [X⁺][SCN⁻] kann durch Einstellen eines Phasengleichgewichts und Trennen der beiden Phasen erfolgen. Darüber hinaus können jedoch in weiteren Ausführungsformen der Erfindung die gängigen, im Stand der Technik beschriebenen Verfahren zur Flüssig-Flüssig-Extraktion angewandt werden. Solche sind beispielsweise Siebbodenkolonnen oder Mixer-Settler- Batterien.

Ferner wurde überraschend gefunden, dass die über das erfindungsgemäße Verfahren hergestellten ionischen Flüssigkeiten zahlreiche außergewöhnliche Eigenschaften besitzen, welche sie für bestimmte technische Anwendungen in der Elektrochemie, bei der Stofftrennung und in chemischen Reaktionen besonders geeignet machen.

Darüber hinaus bietet das neuartige Herstellungsverfahren gemäß dieser Erfindung den überraschenden synthetischen Zugang zu einer großen Zahl weiterer, bisher unbekannten Thiocyanat-Salzen, die wiederum überraschende und technisch interessante Eigenschaften besitzen.

Im Falle der Herstellung einer ionischen Flüssigkeit mit hydrophilem Kation [X⁺][SCN⁻] (Variante 1), werden in Schritt (b) Thiocyanat-Salze [Y⁺][SCN⁻] mit hydrophoben Kationen [Y⁺] verwendet, die ausgewählt sind aus
- Ammoniumthiocyanat-Salzen gemäß der folgenden allgemeinen Formel (I)

   [⁺NR¹R²R³R⁴][SCN⁻] (I)
wobei der Rest R¹ ausgewählt ist aus Wasserstoff, einer linearen oder verzweigten Alkylgruppe mit 1 bis 16, vorzugsweise 1 bis 12 Kohlenstoffatomen und R² bis R⁴ unabhängig voneinander ausgewählt sind aus linearen oder verzweigten Alkylgruppe mit 1 bis 16, vorzugsweise 1 bis 12 Kohlenstoffatomen oder Arylgruppen mit 4 bis 16, vorzugsweise 6 bis 12, besonders bevorzugt 8 bis 10 Kohlenstoffatomen.

Die Reaktion erfolgt in einem Zweiphasensystem umfassend Wasser und ein nicht mit Wasser mischbares organisches Lösungsmittel. Als organische Lösungsmittel werden vorzugsweise lineare oder cyclische Alkyl- oder Arylether, aromatische oder aliphatische Kohlenwasserstoffe, vorzugsweise Diethylether, Tetrahydrofuran (THF), Dibutylether, Toluol, Cyclohexan, Heptan, Chlorbenzol oder deren Mischungen verwendet.

Im Gegensatz dazu werden zur Herstellung von ionischen Flüssigkeiten mit hydrophobem Kation [X⁺][SCN⁻] (Variante 2) in Schritt (b) Thiocyanat-Salze [Y⁺][SCN⁻] mit hydrophilen Kationen [Y⁺] verwendet, die ausgewählt sind aus Alkalimetall-Thiocyanaten, vorzugsweise LiSCN, NaSCN, KSCN oder NH₄SCN.

In diesem Fall erfolgt die Reaktion in einem Zweiphasensystem umfassend Wasser und ein nicht mit Wasser mischbarem organischen Lösungsmittel, welches ausgewählt ist aus Methylenchlorid, Chloroform, Dichlorethan, Trichlorethan oder deren Mischungen.

Im Zusammenhang mit der vorliegenden Erfindung beziehen sich die Begriffe "hydrophob" und "hydrophil" auf die tendenzielle Wassermischbarkeit/-löslichkeit der jeweiligen Salze relativ zueinander. Als "hydrophobe Kationen" werden Kationen bezeichnet, deren Tetrafluoroborat-Salz bei Raumtemperatur (20°C) mit einer gleichen Masse an Wasser versetzt werden kann, ohne dass sich eine homogene, einphasige (vollständige) Lösung des Salzes in Wasser ergibt. Als "hydrophile Kationen" werden dagegen Kationen bezeichnet, deren Tetrafluoroborat-Salz sich bei Raumtemperatur in der gleichen Masse an Wasser unter Bildung einer homogenen, einphasigen Lösung (vollständig) auflöst. Als "hydrophobe Anionen" werden Anionen bezeichnet, deren 1-Butyl-3-methylimidazolium-Salz bei Raumtemperatur mit einer gleichen Masse an Wasser versetzt werden kann, ohne dass sich eine homogene, einphasige (vollständige) Lösung des Salzes in Wasser ergibt. Als "hydrophile Anionen" werden dagegen Anionen bezeichnet, deren 1-Butyl-3-methylimidazolium-Salz sich bei Raumtemperatur in der gleichen Masse an Wasser unter Bildung einer homogenen, einphasigen Lösung (vollständig) auflöst. Neben dieser Definition kann alternativ die weiter unten angegebene Definition über die Gesamtkohlenstoffanzahl zur Beurteilung herangezogen werden.

Das Kation [X⁺] kann im Rahmen der Erfindung ausgewählt sein aus der Gruppe der nachfolgend wiedergegebenen Pyridinium- und Imidazolium-Kationen der Formeln (II) und (III). Die Pyridinium-Kationen der Formel (II) und weisen die nachfolgende Struktur auf: 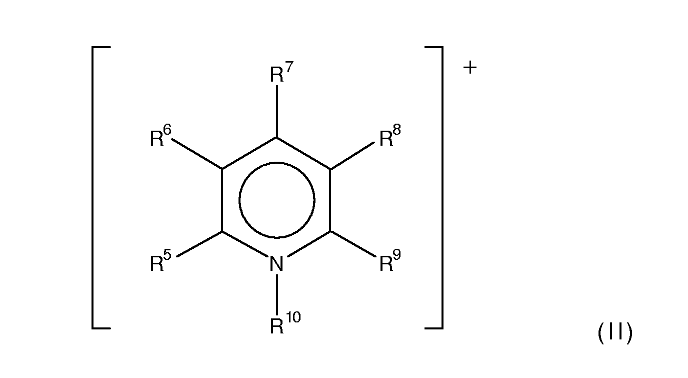 wobei die Reste R⁵ bis R¹⁰ unabhängig voneinander ausgewählt sind aus der Gruppe bestehend aus
- Wasserstoff;
- aliphatischen geradkettigen oder verzweigten Kohlenwasserstoffresten, mit 1 bis 20, vorzugsweise 2 bis 18, besonders bevorzugt 3 bis 14 Kohlenstoffatomen, die in der Kohlenstoffkette zusätzlich Heteroatome, ausgewählt aus N, S und O, sowie Unsättigungen, in Form von einer, zwei oder mehreren konjugierten oder isolierten Doppelbindungen oder einer isolierten Dreifachbindung aufweisen können, wobei gesättigte aliphatische Gruppen bevorzugt sind, wie beispielsweise Kohlenwasserstoffreste, die ausgewählt sind aus der Gruppe bestehend aus Methyl, Ethyl, n-Propyl, Isopropyl, n-Butyl, sec.-Butyl, tert.-Butyl, n-Pentyl, n-Hexyl, n-Octyl oder n-Decyl;
- Cycloaliphatischen Kohlenwasserstoffresten mit 3 bis 20, bevorzugt 5 bis 18, besonders bevorzugt 6 bis 8 Kohlenstoffatomen, wobei die cyclischen Reste Ring-Heteroatome, ausgewählt aus N, S und O, sowie Unsättigungen, in Form von einer, zwei oder mehreren konjugierten oder isolierten Doppelbindungen oder einer isolierten Dreifachbindung aufweisen können;
- Aromatischen und araliphatische Gruppen und mit 5 bis 22, vorzugsweise 6 bis 14, besonders bevorzugt 10 Kohlenstoffatomen im aromatischen Kern, wobei der aromatische Kern 1 bis 4, vorzugsweise 2 bis 3 Heteroatome, ausgewählt aus N, S und O, und lineare oder verzweigte Kohlenwasserstoff- Reste mit 1 bis 10, vorzugsweise 3 bis 8, besonders bevorzugt 4 bis 6 Kohlenstoffatomen aufweisen kann.
   Besonders bevorzugt sind aromatische Reste, die ausgewählt sind aus der Gruppe bestehend aus Phenyl-, Naphthyl- oder Anthracylgruppen.
- Oligoethergruppen Gruppen der allgemeinen Formel (IV)

   -[(CH₂)ₓ-O]_{y}-R¹⁶ (IV),
wobei x und y unabhängig voneinander ausgewählt sind aus ganzen Zahlen zwischen 1 und 250, vorzugsweise 10 und 200, besonders 50 bis 100 und R¹⁶ Wasserstoff oder eine aliphatische, cycloaliphatische, aromatische oder eine araliphatische Gruppe gemäß den vorherigen für R⁵ bis R¹⁰ getroffenen Definitionen darstellt.

Bevorzugte Thiocyanat-Verbindungen weisen Pyridinium-Kationen auf in denen R⁵ bis R¹⁰ Wasserstoff ist oder R¹⁰ ausgewählt ist aus den oben definierten Gruppen der aliphatischen geradkettigen oder verzweigten Kohlenwasserstoffreste, der cycloaliphatischen Kohlenwasserstoffreste, der aromatischen und araliphatische Gruppen und Oligoethergruppen Gruppen der allgemeinen Formel (IV) und R⁵ bis R⁹ Wasserstoff ist.

Besonders bevorzugt sind Pyridinium-Kationen bei denen R¹⁰ ausgewählt ist aus der oben definierten Gruppe der aliphatischen geradkettigen oder verzweigten Kohlenwasserstoffreste und R⁵ bis R⁹ Wasserstoff ist.

Die Imidazolium-Kationen der Formel (III) weisen die nachfolgende Struktur auf: 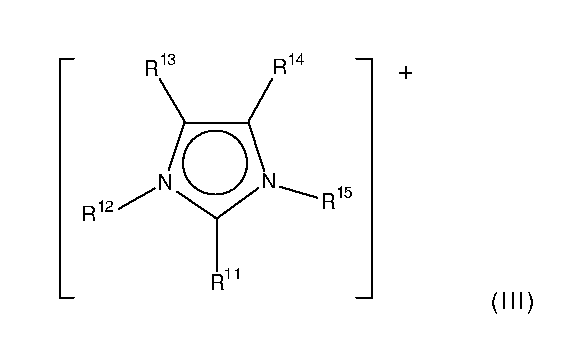 wobei die Reste R¹¹ bis R¹⁵ unabhängig voneinander ausgewählt sind aus der Gruppe bestehend aus
- Wasserstoff;
   - aliphatischen geradkettigen oder verzweigten Kohlenwasserstoffresten, mit 1 bis 20, vorzugsweise 2 bis 18, besonders bevorzugt 3 bis 14 Kohlenstoffatomen, die in der Kohlenstoffkette zusätzlich Heteroatome, ausgewählt aus N, S und O, sowie Unsättigungen, in Form von einer, zwei oder mehreren konjugierten oder isolierten Doppelbindungen oder einer isolierten Dreifachbindung aufweisen können, wobei gesättigte aliphatische Gruppen bevorzugt sind, wie beispielsweise Kohlenwasserstoffreste, die ausgewählt sind aus der Gruppe bestehend aus Methyl, Ethyl, n-Propyl, Isopropyl, n-Butyl, sec.-Butyl, tert.-Butyl, n-Pentyl, n-Hexyl, n-Octyl oder n-Decyl;
- Cycloaliphatischen Kohlenwasserstoffresten mit 3 bis 20, bevorzugt 5 bis 18, besonders bevorzugt 6 bis 8 Kohlenstoffatomen, wobei die cyclischen Reste Ring-Heteroatome, ausgewählt aus N, S und O, sowie Unsättigungen, in Form von einer, zwei oder mehreren konjugierten oder isolierten Doppelbindungen oder einer isolierten Dreifachbindung aufweisen können;
- Aromatischen und araliphatische Gruppen und mit 5 bis 22, vorzugsweise 6 bis 14, besonders bevorzugt 10 Kohlenstoffatomen im aromatischen Kern, wobei der aromatische Kern 1 bis 4, vorzugsweise 2 bis 3 Heteroatome, ausgewählt aus N, S und O, und lineare oder verzweigte Kohlenwasserstoff-Reste mit 1 bis 10, vorzugsweise 3 bis 8, besonders bevorzugt 4 bis 6 Kohlenstoffatomen aufweisen kann.
   Besonders bevorzugt sind aromatische Reste, die ausgewählt sind aus der Gruppe bestehend aus Phenyl-, Naphthyl- oder Anthracylgruppen.
- Oligoethergruppen Gruppen der allgemeinen Formel (IV)

   -[(CH₂)ₓ-O]_{y}-R¹⁶ (IV),
wobei x und y unabhängig voneinander ausgewählt sind aus ganzen Zahlen zwischen 1 und 250, vorzugsweise 10 und 200, besonders 50 bis 100 und R¹⁶ Wasserstoff oder eine aliphatische, cycloaliphatische, aromatische oder eine araliphatische Gruppe gemäß den vorherigen für R¹ bis R¹⁰ getroffenen Definitionen darstellt.

Bevorzugte Thiocyanat-Verbindungen weisen Imidazolium-Kationen auf in denen R¹¹ bis R¹⁵ Wasserstoff ist oder R¹¹, R¹³ und R¹⁴ Wasserstoff ist und R¹² und/oder R¹⁵ unabhängig voneinander ausgewählt sind aus den wie oben definierten Gruppen der aliphatischen geradkettigen oder verzweigten Kohlenwasserstoffreste, der cycloaliphatischen Kohlenwasserstoffreste, der Aromatischen und araliphatische Gruppen und der Oligoethergruppen Gruppen der allgemeinen Formel (IV). Bevorzugt ist, dass wenigstens eine Gruppe R¹² oder R¹⁵ ein wie oben definierter aliphatischer geradkettiger oder verzweigter Kohlenwasserstoffrest ist

Bevorzugte Thiocyanat-Verbindungen weisen Imidazolium-Kationen auf in denen R¹¹, R¹³ und R¹⁴ wie oben definiert sind und in denen R¹² und R¹⁵, neben den oben für die aliphatischen geradkettigen oder verzweigten Kohlenwasserstoffresten mit 3 bis 20, vorzugsweise 3 bis 18, besonders bevorzugt 3 bis 14, die folgenden Bedeutungen haben kann:
- R¹² und R¹⁵ jeweils beide Wasserstoff, Methyl oder Ethyl; oder
- R¹² oder R¹⁵ Methyl oder Ethyl und R¹⁵ oder R¹² Wasserstoff ist.

Hierdurch ist die von Golding *et al* beschriebene, jedoch durch eine gegenüber einer vergleichbaren Verbindung (1,3-Dimethylimidazoliumthiocyanat) durch eine höhere Viskosität und eine geringere elektrische Leitfähigkeit gekennzeichnete Verbindung (1-Ethyl-3-methylimidazoliumthiocyanats) ausdrücklich von den ionischen Flüssigkeiten als Stoffen dieser Erfindung ausgenommen.

Unabhängig von der oben beschriebenen Definition kann eine Unterscheidung oder Abgrenzung zwischen hydrophilem und hydrophobem Kation [X⁺] kann dahingehend getroffen werden, dass wenn die Gesamtsumme der Kohlenstoffatome in den Gruppen R⁵ bis R¹⁰ (Pyridinium-Kation) und R¹¹ bis R¹⁵ (Imidazolium-Kation) von 0 bis 5, vorzugsweise bis 4 ist, es sich um hydrophile Kationen [X⁺] und bei einer Gesamtsumme der Kohlenstoffatome in den Gruppen R⁵ bis R¹⁰ und R¹¹ bis R¹⁵ von wenigstens 6, vorzugsweise wenigstens 7 es sich um hydrophobe Kationen [X⁺] handelt.

Typische Beispiele für hydrophile Kationen [X⁺] sind Pyridinium-, Imidazolium-, 1-Methoxyethylpyridinium-, 1 -Ethoxyethylpyridinium-, 1 -Methyl-3-methoxyethylimidazolium-, 1-Methyl-3-ethoxyethylimidazolium-, 1-Methylpyridinium-, 1-Ethylpyridinium-, 1-Propylpyridinium-, 1-Butylpyridinium-, 1-Methoxyethylpyridinium-, 1-Ethoxyethylpyridinium-, 1- oder 3-Methylimidazolium-, 1- oder 3-Ethylimidazolium-, 1,3-Dimethylimidazolium-, 1-Ethyl-3-methylimidazolium-, 1,3-Diethylimidazolium-, 1-Methyl-3-propylimidazolium-, 1-Butyl-3-methylimidazolium-, 1,2,3-Trimethylimidazolium-, 1-Ethyl-2,3-Dimethylimidazolium- und 1,1-Dimethylpyrrolidinium-Kationen.

Typische Beispiele für hydrophobe Kationen [X⁺] sind hingegen 1-Hexyl-3-methylimidazolium-, 1-Methyl-3-octylimidazolium-, 1-Decyl-3-methylimidazolium-, 1-Hexylpyridinium-, 1-Octylpyridinium-, 1-Decylpyridinium-, 1-Hexyl-2,3-dimethylimidazolium-, 1-Octyl-2,3-dimethylimidazolium-, 1-Decyl-2,3-dimethylimidazolium- und 1-Dodecyl-2,3-dimethylimidazolium-Kationen.

Typische Beispiele für die in Schritt (a) verwendeten hydrophoben Anionen [A⁻] sind Bis-trifluorosulfonimid ((CF₃SO₂)₂N⁻), Hexafluorophosphat (PF₆⁻), Trifluorotris(pentafluoroethyl)phosphat [PF₃(CF₃CF₂)₃]⁻ und Nonafluorobutansulfonat [F₃C-(CF₂)₃SO₃]⁻.

Typische Beispiele für die in Schritt (a) verwendeten hydrophilen Anionen [A⁻] sind Tosylate (CH₃PhSO₃⁻); Methylsulfat (MeSO₄⁻); Methylsulfonat (MeSO₃⁻); Ethylsulfat (EtSO₄⁻); Ethylsulfonat (EtSO₃⁻); Butylsulfat (BuSO₄⁻⁾; Hexylsulfat (HexSO₄⁻) Octylsulfat (OctylSO₄⁻); Sulfat, Phosphat, Nitrat, Nitrit und Hydrogensulfat.

Die Herstellung eines Thiocyanat-Salzes [X⁺][SCN⁻] mit hydrophilem Kation soll nachfolgend am Beispiel der Herstellung von 1 -Ethyl-3-methylimidazolium-Thiocyanat ([EMIM⁺][SCN⁻]) erläutert werden. Das Verfahren gemäß dieser Erfindung (gemäß Variante 1) umfasst folgende Schritte:
a) Herstellung von [EMIM⁺][(CF₃SO₂)₂N⁻] ((P. Bonhöte, A.-P. Dias, N. Papageorgiou, K. Kalyanasundaram, M. Grätzel, Inorg. Chem. 1996, 35, 1168 - 1178), [EMIM][PF₆] (R. T. Fuller, H. C. Carlin, H. C. de Long, D. Haworth, J. Chem. Soc. Chem. Commun. 1994, 299 - 300) oder ein anderes [EMIM⁺]-Salz eines hydrophoben Anions nach im Stand der Technik beschriebenen Verfahren;
b) Umsetzen des in Schritt (a) erhaltenen Salzes mit
   - einem bekanntem Ammoniumthiocyanat-Salz gemäß der folgenden allgemeinen Formel (I)

      [⁺NR¹R²R³R⁴][SCN⁻] (I)
   wobei der Rest R¹ ausgewählt ist aus Wasserstoff oder einer linearen oder verzweigten Alkylgruppe mit 1 bis 16, vorzugsweise 1 bis 12 Kohlenstoffatomen und R² bis R⁴ unabhängig voneinander ausgewählt sind aus linearen oder verzweigten Alkylgruppen mit 1 bis 16, vorzugsweise 1 bis 12 Kohlenstoffatomen oder Arylgruppen mit 5 oder 6 bis 16, vorzugsweise 6 bis 12, besonders bevorzugt 8 bis 10 Kohlenstoffatomen,
   unter Rühren in einem Wasser/organischen Lösungsmittel Zweiphasen-System, wie oben beschrieben, vorzugsweise in einem Zweiphasen-System aus Diethylether und Wasser;
(c) Einstellung eines stabilen Phasengleichgewichts und Trennung der beiden Phasen.

Die Wasserphase enthält [ EMIM⁺] [SCN⁻] in hochreiner Form, während die Etherphase die Kombination der eingesetzten hydrophoben Ionen, z.B. [⁺NR¹R²R³R⁴][(CF₃SO₂)₂N⁻] oder [⁺NR¹R²R³R⁴][PF₆⁻] enthält. Das Produkt kann abschließend durch destillatives Entfernen des Wassers isoliert werden. Vorzugsweise wird die destillative Entfernung des Wassers unter reduziertem Druck durchgeführt.

In einer weiteren bevorzugten Ausführungsform des erfindungsgemäßen Verfahrens wird [EMIM⁺][(CF₃SO₂)₂N⁻] mit [⁺N(n-Bu)₄] in ein Gemisch aus Diethylether und Wasser gegeben und nach Phasentrennung und Entfernen des Wassers [EMIM⁺][SCN⁻] isoliert. Das in der Etherphase isolierte [⁺N(n-Bu)₄][(CF₃SO₂)₂N⁻] stellt selbst eine ionische Flüssigkeit dar, die sich überraschenderweise nicht mit [EMIM⁺][SCN⁻] mischt. Soll eine Wiederverwendung des [(CF₃SO₂)₂N⁻]-Anions für die erneute Synthese einer neuen Charge einer ionischen Flüssigkeit gemäß dieser Erfindung erfolgen, so kann das in der Etherphase isolierte [⁺N(n-Bu)₄][(CF₃SO₂)₂N⁻] mit einer basischen wässrigen Lösung versetzt werden und unter Rückfluss zwischen 2 bis 5 h, vorzugsweise 3 h erhitzt werden. Schließlich kann das Amin durch Extraktion mit Diethylether abgetrennt werden. Die wässrige Lösung des [(CF₃SO₂)₂N⁻]-Anions wird neutral gestellt (pH = etwa 7) und kann so erneut zur Synthese des [EMIM⁺][(CF₃SO₂)₂N⁻] eingesetzt werden.

In weiteren erfindungsgemäßen Ausführungsform der Variante 1 kann Diethylether durch andere lineare oder cyclische Alkyl- oder Arylether sowie durch aromatische oder aliphatische Kohlenwasserstoffe ersetzt werden. Besonders geeignete Lösungsmittelalternativen zu Diethylether umfassen THF, Dibutylether, Toluol, Cyclohexan, Heptan oder Chlorbenzol.

Die Herstellung eines Thiocyanat-Salzes mit hydrophoben Kationen (Variante 2) soll anhand der Herstellung von N-Hexyl-N,N,N-tributylthiocyanat näher beispielhaft erläutert werden. Das erfindungsgemäße Verfahren umfasst die folgenden Schritte:
(a) Herstellung eines Salzes, z.B. N-Hexyl-N,N,N-tributyltosylat, umfassend das gewünschte Kation und ein hydrophiles Anion nach einem Verfahren des Standes der Technik (beispielsweise durch Umsetzung von Toluolsulfonsäurehexylester mit Tributylamin),
(b) Umsetzen des in Schritt (a) erhaltenen Salzes mit einem Alkalimetall-, vorzugsweise LiSCN, NaSCN, KSCN oder NH₄SCN in einem Gemisch aus Methylenchlorid und Wasser;
(c) Einstellung eines stabilen Phasengleichgewichts und Trennung der beiden Phasen.

Die Wasserphase enthält das Alkalimetall- oder Ammoniumsalz [Y⁺][A⁻] (hier z.B. Li-, Na-, K-, oder NH₄-tosylat) des hydrophilen Anions des Zwischenproduktes aus a) während die ionische Flüssigkeit [X⁺][SCN⁻] in hochreiner Form aus der Methylenchlorid-Phase durch Verdampfen des Lösungsmittels isoliert werden kann. Vorzugsweise wird die destillative Entfernung des Methylenchlorids unter reduziertem Druck durchgeführt.

In weiteren erfindungsgemäßen Ausführungsformen der Variante 2 kann Methylenchlorid beispielsweise durch Chloroform, Dichlorethan oder Trichlorethan ersetzt werden.

Für den Fachmann liegen die Vorteile des extraktiven Verfahrens gemäß dieser Erfindung auf der Hand: Als Quelle des Thiocyanat-Anions wird im Verfahren gemäß dieser Erfindung anstelle des von Golding *et al* verwendeten extrem teuren Silbersalzes die weitaus preiswerteren Tetraalkylammonium-Thiocyanate (Variante 1) bzw. die Alkalimetall- oder NH₄-Thiocyanate (Variante 2) eingesetzt.

Wesentlich wichtiger in Bezug auf die Verwendung der Thiocyanate in Anwendungen der Elektrochemie, bei der Stofftrennung und chemischen Reaktionen ist aber die Tatsache, dass die nach dem erfindungsgemäßen Verfahren hergestellten ionischen Flüssigkeiten absolut frei sind von lodid und Ag⁺-Verunreinigungen. Unter "absolut frei" sind hierbei jeweils Ag⁺ und l⁻ Konzentrationen von < 5 ppm, vorzugsweise < 3 ppm zu verstehen.

Diese wesentlich höhere Reinheit hat direkte Auswirkungen auf die anwendungsrelevanten physiko-chemischen Eigenschaften der erhaltenen ionischen Flüssigkeiten. Besonders offensichtlich ist die bedeutend höhere Lichtstabilität der nach dem neuen Verfahren erhaltenen ionischen Flüssigkeiten. Während sich die nach Golding *et al* hergestellten ionischen Flüssigkeiten bereits nach wenigen Stunden dunkel verfärben und schließlich unter Licht deutlich auch ihre physikalisch-chemischen Eigenschaften verändern, konnten die nach dem erfindungsgemäßen Verfahren hergestellten ionischen Flüssigkeiten mehrere Wochen ohne Farbveränderung oder sonstige Veränderung ihrer Eigenschaften an Licht gelagert werden. Dabei gab es auch keinerlei Hinweis darauf, dass eine längere Lagerung an Licht zu irgendeiner Beeinträchtigung der Qualität der ionischen Flüssigkeit führen könnte. Die Erhöhung der Lichtstabilität durch das neue Herstellverfahren ist eine wesentliche Voraussetzung für die Verwendung der ionischen Flüssigkeiten mit Thiocyanat-Ion in Anwendungen unter Lichteinfluss, wie z.B. der Verwendung als Elektrolyt in Farbstoffsolarzellen, aber auch in allen anderen Anwendungen von großer Bedeutung, in denen ein Lichtausschluss nicht gewährleistet werden kann.

Im Vergleich zu den von Golding *et al* veröffentlichten Eigenschaften fällt außerdem die höhere thermische und elektrochemische Stabilität der nach dem erfindungsgemäßen Verfahren hergestellten Substanzen, insbesondere unter anwendungstechnischen Gesichtspunkten ins Gewicht.

Weitere Eigenschaften der hergestellten ionischen Flüssigkeiten wurden von Golding *et al* auf Grund der mangelnden Qualität der erhaltenen Substanzen gar nicht untersucht, können nun aber mit den hochreinen Substanzen, die gemäß dem Verfahren dieser Erfindung hergestellt werden können, ermittelt werden. Überraschenderweise wurde festgestellt, dass die Substanzen in hochreiner Form eine ungewöhnlich niedrige Viskosität und eine außergewöhnlich hohe elektrische Leitfähigkeit besitzen (siehe Beispiele). Genau diese Eigenschaftskombination ist für die Anwendungen im Bereich der Elektrochemie, der extraktiven Stofftrennung und der Mehrphasenkatalyse von entscheidender Bedeutung.

Ferner eröffnet die neuartige Herstellungsmethode gemäß dieser Erfindung Zugang zu zahlreichen neuen und bisher nicht beschriebenen Thiocyanat-Salze, die sogar noch wesentlich interessantere Eigenschaften für zahlreiche Anwendungen bieten.

Die Erfindung betrifft außerdem ionische Flüssigkeiten der Formel [X⁺][SCN⁻], wobei das Kation [X⁺] die oben angegebenen Bedeutungen aufweist. Zu diesem Zwecke wird ausdrücklich auf die obigen Definitionen Bezug genommen.

Bei den ionischen Flüssigkeiten gemäß der vorliegenden Erfindung handelt es sich um Substanzen deren Eigenschaften sich wesentlich von denen des von Golding *et al* hergestellten 1-Ethyl-3-methylimidazoliumthiocyanats unterscheiden, wie an einem Beispiel einer ionischen Flüssigkeit gemäß dieser Erfindung, dem 1,3-Dimethylimidazoliumthiocyanat, deutlich wird.

Im Gegensatz zu ionischen Flüssigkeiten mit dem [(CF₃SO₂)₂N⁻]-Anion, liegt die Viskosität des 1,3-Dimethylimidazoliumthiocyanats ([MMIM⁺][SCN⁻]) deutlich über der des entsprechenden 1 -Ethyl-3-methylimidazoliumthiocyanats ([EMIM⁺][SCN⁻]) (Werte für die ionischen Flüssigkeiten mit [(CF₃SO₂)₂N⁻]-Ion: [MMIM⁺][SCN⁻]= 0,044 Pa s (44 cP) gegenüber [EMIM⁺][(CF₃SO₂)₂N⁻]= 0,034 Pa s (34 cP) gemäß P. Bonhöte, A.-P. Dias, N. Papageorgiou, K. Kalyanasundaram, M. Grätzel, Inorg. Chem. 1996, 35, 1168-1178). Es wurde überraschenderweise gefunden, dass die neuartige ionische Flüssigkeit gemäß dieser Erfindung [MMIM⁺][SCN⁻] mit 0,0207 Pa s (20,7 cP) (bei 230 ppm Wassergehalt) eine deutlich niedrigere Viskosität aufweist als die von Golding *et al* beschriebene ionische Flüssigkeit [EMIM⁺][SCN⁻]. Ferner zeichnet sich [MMIM⁺][SCN⁻] durch eine extrem hohe elektrische Leitfähigkeit von 20 mS/cm bei 25 °C aus. Damit besitzt diese ionische Flüssigkeit bei weitem die höchste elektrische Leitfähigkeit aller nicht-Chloroaluminat-Schmelzen. Außerdem zeichnet sich [MMIM⁺][SCN⁻] durch eine überraschend hohe Schmelzenthalphie von über 200 J/g aus. Die Kombination aus niedriger Viskosität und hoher Leitfähigkeit ist für Anwendungen in der Stofftrennung, der Mehrphasenkatalyse, vor allem aber bei der Elektrochemie von hohem technischen Wert.

Die Tatsache, dass durch Variation der Reste R⁵ bis R¹⁵ eine Vielzahl ionischer Flüssigkeiten mit technisch-hochinteressanten Eigenschaften zur Verfügung gestellt werden können, wird durch zahlreiche weitere ionische Flüssigkeiten gemäß dieser Erfindung gestützt.

Nachfolgend sind einige besonders bevorzugte ionische Flüssigkeiten gemäß dieser Erfindung aufgeführt, wobei die bevorzugte Herstellungsvariante in Klammer angegeben ist:
Imidazoliumthiocyanat (nach Variante 1)
Pyridiniumthiocyanat (nach Variante 1)
1,3-Dimethylimidazoliumthiocyanat (nach Variante 1)
1- und 3-Methylimidazoliumthiocyanat (nach Variante 1)
1 ,3-Diethylimidazoliumthiocyanat (nach Variante 1)
1-und 3-Ethylimidazoliumthiocyanat (nach Variante 1)
1-Methyl-3-propylimidazoliumthiocyanat (nach Variante 1)
1-Butyl-3-methylimidazoliumthiocyanat (nach Variante 1 und 2)
1-Hexyl-3-methylimidazoliumthiocyanat (nach Variante 2)
1-Methyl-3-octylimidazoliumthiocyanat (nach Variante 2)
1-Decyl-3-methylimidazoliumthiocyanat (nach Variante 2)
1 -Methyl-3-methoxyethylimidazoliumthiocyanat (nach Variante 1)
1-Methyl-3-ethoxyethylimidazoliumthiocyanat (nach Variante 1)
1-Methylpyridiniumthiocyanat (nach Variante 1)
1-Ethylpyridiniumthiocyanat (nach Variante 1)
1-Propylpyridiniumthiocyanat (nach Variante 1)
1 - -Butylpyridiniumthiocyanat (nach Variante 1)
1-Hexylpyridiniumthiocyanat (nach Variante 2)
1-Octylpyridiniumthiocyanat (nach Variante 2)
1-Decylpyridiniumthiocyanat (nach Variante 2)
1-Methyoxyethylpyridiniumthiocyanat (nach Variante 1)
1-Ethoxyethylpyridiniumthiocyanat (nach Variante 1).

Die erfindungsgemäß hergestellten ionischen Flüssigkeiten können einer Vielzahl von Verwendungen zugeführt werden. So eignen sie sich aufgrund ihrer Reinheit, des nicht vorhandenen Dampfdrucks und der hohen Beständigkeit als Lösungsmittel oder Lösungsmittelzusatz in chemischen Reaktionen. Die Verwendung als Lösungsmittelzusatz erfolgt in Kombination mit anderen Lösungsmitteln. Des Weiteren können die erfindungsgemäßen ionischen Flüssigkeiten als Phasentransferkatalysatoren, in mehrphasigen Reaktionssystemen als oberflächenaktive Substanzen oder Weichmacher eingesetzt werden. Außerdem eignen sich die erfindungsgemäßen ionischen Flüssigkeiten als Extraktionsmittel in Stofftrennverfahren, als Elektrolyt in Batterien oder Kondensatoren oder als Bestandteil einer Farbstoffsolarzelle oder eines Sensors.

Die vorliegende Erfindung soll anhand der folgenden Beispiele näher erläutert werden, ohne sie jedoch auf die Beispiele zu beschränken.

### Beispiele

Die Viskositäten wurden auf dem Gerät RS 100 RheoStress der Firma HAAKE im CR-Modus (Controlled Rate) gemessen. Viskositäten wurden bei einer Temperatur von 25°C bestimmt.

Die Leitfähigkeiten wurden auf dem Gerät der Firma WTW Typ LF 530, das mit der Messelektrode 980-K19/120 der Firma METTLER-TOLEDO bestückt war gemessen. Alle Messungen wurden bei 22°C durchgeführt.

### Beispiel 1: Herstellung von 1-Ethyl-3-methylimidazolium-Thiocyanat ([EMIM⁺][SCN⁻] gemäß erfindungsgemäßer Variante 1

Synthese: 5,05 g 1-Ethyl-3-methylimidazoliumhexafluorophosphat ([EMIM⁺][PF₆⁻]) (19,72mmol) und 5,93 g Tetrabutylammonium-Thiocyanat (19,72 mmol) werden in ein Gemisch aus 50 ml Wasser und 50 ml Diethylether gegeben. Es bildet sich Tetrabutylammoniumhexafluorophosphat als unlöslicher Feststoff der abfiltriert wird. Das Filtrat wird erneut zweimal mit je 50 ml Wasser extrahiert. Die gesammelten Wasserphasen werden isoliert und eingeengt. Man erhält 2,00 g 1-Ethyl-3-methylimidazoliumrhodanid als schwach-gelbe, niedrig-viskose Flüssigkeit (60% Ausbeute). Der Gehalt an Ag⁺ und I⁻-Ionen im Produkt liegt jeweils unter 3 ppm.
Weitere Eigenschaften:
Viskosität: 0,0243 Pa s (24,3 cP) [bei 25°C und einem Wassergehalt (Karl-Fischer-Titration) von 235 ppm].

Die Probe ist lichtstabil, d.h. es erfolgt über 3 Wochen keine Verfärbung an Licht.
Elektrische Leitfähigkeit: 12 mS/cm
NMR-Daten:
¹H-NMR(300 MHz, CDCl₃): 9,03 ppm (s, NCHN); 7,70 ppm; 7,69 ppm (2s, CH); 4,26 ppm (q, N-CH₂); 4,02 ppm (s, CH₃); 1,42 ppm (t, CH₂-CH₃). ¹³C-NMR (75 MHz, CDCl₃): 14,8 ppm (CH₂-CH₃); 35,7 ppm (N-CH₃); 44,5 ppm (CH₂-CH₃); 121,5 ppm; 123,0 ppm (CH); 130,1 ppm (SCN); 135,7 ppm (NCHN).

### Vergleichsbeispiel 1: Herstellung von 1-Ethyl-3-methylimidazolium-Thiocyanat ([EMIM⁺][SCN⁻]) gemäß der Literaturvorschrift von Golding et al

1,91 g AgSCN (11 mmol) wird durch Vermischen wässriger Lösungen von gleichen Mengen an Silbernitrat und KSCN erhalten, gut mit Wasser gewaschen und sofort weiterverwendet. Eine wässrige Lösung von 2,61 g 1-Ethyl-3-methylimidazoliumiodid ([EMIM]I) (11 mmol) wird zu der Suspension von AgSCN gegeben und die Lösung unter leichtem Erhitzen eine Stunde gerührt. Das gebildete Silberiodid wird filtriert und das Wasser am Vakuum entfernt. Um alle Silbersalze zu entfernen wird das Produkt mit Acetonitril verdünnt und einen Tag im Gefrierschrank gekühlt. Der so gebildete Niederschlag wird filtriert und das Lösungsmittel entfernt. Das Produkt wird in 80% Ausbeute erhalten. Es enthält jeweils 5000 ppm Ag⁺ und 5000 ppm I⁻ - Ionen.

Unter Lichteinwirkung wird das so erhaltene Produkt schnell (innerhalb weniger Stunden) grau-schwarz und die physikalisch-chemischen Eigenschaften der Schmelze verändern sich in offensichtlicher Weise.

### Beispiel 2: Herstellung von N-Hexyl-N,N,N-Tributylammonium-Thiocyanat nach dem Verfahren der Erfindung

### Synthese:

0,5 mol Tributylamin wird bei 80 °C zu 0,5 mol Toluolsulfonsäurehexylester getropft und die Mischung wird 4 Stunden auf 80 °C erhitzt. Das abgekühlte Rohprodukt wird in 300 ml Wasser gelöst, mit 0,5 mol NH₄SCN versetzt und dreimal mit 300 ml CH₂Cl₂ extrahiert. Die vereinigten Methylenchlorid-Extrakte werden getrocknet und eingeengt. Der Gehalt an Ag⁺ und I⁻ im Produkt liegt jeweils unter 3 ppm.

Das Produkt wird als schwach-gelbe Flüssigkeit erhalten (Ausbeute 80 %) und verfärbt sich auch nach 3 Wochen unter Lichteinfluss nicht.
NMR-Daten:
¹H-NMR (300 MHz, d⁶-DMSO): 3,15 ppm (3x NCH₂); 3,05 ppm (NCH₂); 1,56 ppm (4xCH₂); 1,22 ppm (4xCH₂);0,85 ppm (4xCH₃).

### Vergleichsbeispiel 2: Herstellung von N-Hexyl-N,N,N-Tributylammonium-Thiocyanat gemäß der Literaturvorschrift von Golding et al

1,91 g AgSCN (11 mmol) wird durch Vermischen wässriger Lösungen von gleichen Mengen an Silbernitrat und KSCN erhalten, gut mit Wasser gewaschen und sofort weiterverwendet. Eine wässrige Lösung von 4,35 g N-Hexyl-N,N,N-tributylammoniumiodid (11 mmol) wird zu der Suspension von AgSCN gegeben und die Lösung unter leichtem Erhitzen eine Stunde gerührt. Das gebildete Silberiodid wird filtriert und das Wasser am Vakuum entfernt. Um alle Silbersalze zu entfernen wird das Produkt mit Acetonitril verdünnt und einen Tag im Gefrierschrank gekühlt. Der so gebildete Niederschlag wird filtriert und das Lösungsmittel entfernt. Das Produkt wird in 97 % Ausbeute erhalten. Es enthält jeweils 5000 ppm Ag⁺ und 5000 ppm I⁻- Ionen.

Unter Lichteinwirkung wird das so erhaltene Produkt schnell (innerhalb weniger Stunden) grau-schwarz und die physikalisch-chemischen Eigenschaften der Schmelze verändern sich in offensichtlicher Weise.

### Beispiel 3: Synthese von 1,3-Dimethylimidazolium-Thiocyanat (nach Variante 1)

Synthese: 79,25 g 1,3-Dimethylimidazoliumbistrifluorosulfonimid ([MMIM⁺]-[(CF₃SO₂)₂N⁻]) (210 mmol) und 63,13 g Tetrabutylammonium-Thiocyanat (210 mmol) werden in ein Gemisch aus 200 ml Wasser und 200 ml Diethylether gegeben. Das Gemisch wird intensiv durchmischt und die Phasen werden getrennt. Während sich Tetrabutylammoniumbistrifluorosulfonimid selektiv in der Etherphase löst, wird das Produkt in reiner Form in die Wasserphase extrahiert. Die Wasserphase wird am Vakuum eingeengt. Man erhält 105 mmol des Produkts 1,3-Dimethylimidazoliumthiocyanat in Form einer sehr niedrigviskosen, leicht gelblichen Flüssigkeit (50% Ausbeute). Der Gehalt an Ag⁺ und I⁻ im Produkt liegt unter 3 ppm.
Weitere Eigenschaften:
Schmelzpunkt: 13°C
Viskosität: 0,0207 Pa s (20,7 cP) [bei 25°C und einem Wassergehalt (Karl-Fischer-Titration) von 273 ppm].

Probe ist lichtstabil (keine Verfärbung an Licht) über 3 Wochen.
Elektrische Leitfähigkeit: 20 mS/cm
NMR-Daten:
¹H-NMR(300 MHz, CD₃CN): 8,79 ppm (s, NCHN); 7,47 ppm; (s, CH); 3,86 ppm (s, N-CH₃).
¹³C-NMR (75 MHz, CDCl₃): 35,3 ppm (NCH₃); 121,5 ppm; 123,0 ppm (CH); 130,1 ppm (SCN); 135,7 ppm (NCHN).

### Beispiel 4: Synthese von 1-Ethylpyridinium-Thiocyanat ([Et-Py⁺][SCN⁻])(nach Variante 1)

### Synthese:

14,35 g 1-Ethylpyridiniumbistrifluorosulfonimide ([Et-Py⁺][(CF₃SO₂)₂N⁻]) (36,95 mmol) und 15,25 g Trioctylammoniumrhodanid (36,95 mmol) werden in ein Gemisch aus 80 ml Wasser und 80 ml Diethylether gegeben. Das Gemisch wird intensiv durchmischt und die Phasen werden getrennt. Während sich Trioctylammoniumbistrifluorosulfonimid selektiv in der Etherphase löst, wird das Produkt in reiner Form in die Wasserphase extrahiert. Die Wasserphase wird am Vakuum eingeengt. Man erhält das Produkts 1-Ethylpyridiniumthiocyanat in 45 % Ausbeute in Form einer sehr niedrig viskosen, rötlich gefärbten Flüssigkeit. Der Gehalt an Ag⁺ und I⁻ im Produkt liegt jeweils unter 3 ppm.
Weitere Eigenschaften:
Viskosität: 0,0291 Pa s (29,1 cP) [bei 25°C und einem Wassergehalt (Karl-Fischer-Titration) von 3900 ppm].
Probe ist lichtstabil (keine Verfärbung an Licht) über 3 Wochen.
NMR-Daten:
¹H-NMR (300 MHz, D₂O): 8,80 ppm (NCH); 8,54 ppm; (CH); 8,08 (N-CH-CH); 4,68 ppm (NCH₂); 1,65 ppm (NCH₂-CH₃).

### Beispiel 5: Synthese von 1-Methyl-3-octylimidazolium-Thiocyanat ([BMIM⁺][SCN⁻]) (nach Variante 2)

### Synthese:

9,89 g 1-Methyl-3-octylimidazoliumhydrogensulfat (33,8 mmol) werden in 40 ml Wasser gelöst und 33,8 mmol NaOH zugegeben. Die Lösung wird 15 min bei Raumtemperatur gerührt. 2,57 g NH₄SCN (33,8 mmol) wird zugegeben und wieder 15 min. bei Raumtemperatur gerührt. Die wässrige Lösung wird dreimal mit 50 ml Methylenchlorid extrahiert, die Methylenchlorid-Phasen getrocknet und am Vakuum eingeengt. Die Ausbeute des Produkts beträgt 90 %. Der Gehalt an Ag⁺ und I⁻ - Ionen im Produkt liegt jeweils unter 3 ppm. Außerdem ist das Produkt über Wochen hinweg gegen Lichteinwirkung stabil.
NMR-Daten:
¹³C-NMR (75 MHz, CDCl₃): 14,2 ppm; 22,9 ppm; 26,5 ppm; 29,2 ppm; 29,3 ppm; 30,5 ppm; 32,0 ppm; 36,9 ppm (C-alkyl); 50,5 ppm (NCH₃); 121,5 ppm; 123,0 ppm (CH); 130,1 ppm (SCN); 135,7 ppm (NCHN).
Weitere Eigenschaften:
Viskosität: 1,554 Pa s (155,4 cP) [bei 25°C und einem Wassergehalt (Karl-Fischer-Titration) von 320 ppm].

## Patentansprüche

1. Verfahren zur Herstellung von ionischen Flüssigkeiten der allgemeinen Formel [X⁺][SCN⁻], wobei X⁺ ein hydrophiles oder hydrophobes Kation ist, umfassend die Schritte
(a) die Herstellung eines Salzes [X⁺][A⁻];
(b) das Umsetzen des in Schritt (a) erhaltenen Salzes [X⁺][A⁻] mit einem Thiocyanat-Salz [Y⁺][SCN⁻] in einem Zweiphasensystem aus Wasser und einem nicht mit Wasser mischbaren organischen Lösungsmittel;
(c) die Isolierung der ionischen Flüssigkeit [X⁺][SCN⁻] aus einer der beiden Phasen, wobei
- im Falle eines hydrophilen Kations [X⁺] in Schritt (a) ein Salz [X⁺][A⁻] mit einem hydrophoben Anion [A⁻] hergestellt wird, welches in Schritt (b) mit einem Thiocyanat-Salz [Y⁺][SCN⁻] mit hydrophobem Kation [Y⁺] umgesetzt wird, und
- im Falle eines hydrophoben Kations [X⁺] in Schritt (a) ein Salz [X⁺][A⁻] mit einem hydrophilen Anion [A⁻] hergestellt wird, welches in Schritt (b) mit einem Thiocyanat-Salz [Y⁺][SCN⁻] mit hydrophilen Kation [Y⁺] umgesetzt wird.

2. Verfahren gemäß Anspruch 1, **dadurch gekennzeichnet, dass** das Thiocyanat-Salz [Y⁺][SCN⁻] mit dem hydrophobem Kation [Y⁺] ausgewählt ist aus
- Ammoniumthiocyanat-Salzen gemäß der folgenden allgemeinen Formel (I)
[⁺NR¹R²R³R⁴][SCN⁻] (I)
wobei der Rest R¹ ausgewählt ist aus Wasserstoff, einer linearen oder verzweigten Alkylgruppe mit 1 bis 16, vorzugsweise 1 bis 12 Kohlenstoffatomen und R² bis R⁴ unabhängig voneinander ausgewählt sind aus linearen oder verzweigten Alkylgruppe mit 1 bis 16, vorzugsweise 1 bis 12 Kohlenstoffatomen oder Arylgruppen mit 5 oder 6 bis 16, vorzugsweise 6 bis 12, besonders bevorzugt 8 bis 10 Kohlenstoffatomen.

3. Verfahren gemäß Anspruch 1, wobei das Thiocyanat-Salz [Y⁺][SCN⁻] mit dem hydrophilen Kation [Y⁺] ein Alkalimetall- oder NH₄⁺-Thiocyanat ist.

4. Verfahren gemäß irgendeinem der Ansprüche 1 oder 2, wobei das organische Lösungsmittel ausgewählt ist aus der Gruppe bestehend aus linearen oder cyclischen Alkyl- oder Arylethern, aromatischen oder aliphatischen Kohlenwasserstoffen oder deren Mischungen, vorzugsweise Diethylether, THF, Dibutylether, Toluol, Cyclohexan, Heptan, Chlorbenzol oder deren Mischungen verwendet werden.

5. Verfahren gemäß irgendeinem der Ansprüche 1 oder 3, wobei das organische Lösungsmittel ausgewählt ist aus der Gruppe bestehend aus Methylenchlorid, Chloroform, Dichlorethan, Trichlorethan oder deren Mischungen.

6. Verfahren gemäß irgendeinem der Ansprüche 1 bis 5, **dadurch gekennzeichnet, dass** das Kation [X⁺] wie folgt definiert ist: 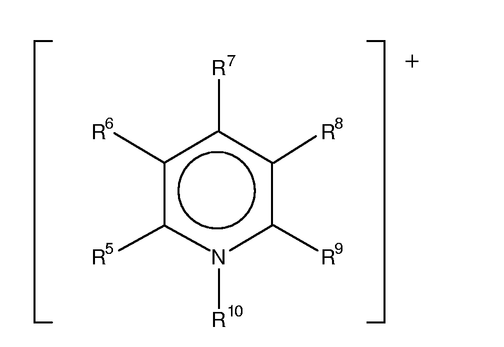 wobei die Reste R⁵ bis R¹⁰ unabhängig voneinander ausgewählt sind aus der Gruppe bestehend aus
- aliphatischen geradkettigen oder verzweigten Kohlenwasserstoffresten, mit 1 bis 20, vorzugsweise 2 bis 18, besonders bevorzugt 3 bis 14 Kohlenstoffatomen, die in der Kohlenstoffkette zusätzlich Heteroatome, ausgewählt aus N, S und O, sowie Unsättigungen, in Form von einer, zwei oder mehreren konjugierten oder isolierten Doppelbindungen oder einer isolierten Dreifachbindung aufweisen können, wobei gesättigte aliphatische Gruppen bevorzugt sind,
- cycloaliphatischen Kohlenwasserstoffresten mit 3 bis 20, bevorzugt 5 bis 18, besonders bevorzugt 6 bis 8 Kohlenstoffatomen, wobei die cyclischen Reste Ring-Heteroatome, ausgewählt aus N, S und O, sowie Unsättigungen, in Form von einer oder mehreren konjugierten oder isolierten Doppelbindungen oder einer isolierten Dreifachbindung aufweisen können,
- aromatischen und araliphatischen Gruppen mit 5 oder 6 bis 22, vorzugsweise 6 bis 14, besonders bevorzugt bis 10 Kohlenstoffatomen im aromatischen Kern, wobei der aromatische Kern 1 bis 4, vorzugsweise 2 bis 3 Heteroatome, ausgewählt aus N, S und O, und lineare oder verzweigte Kohlenwasserstoff-Reste mit 1 bis 10, vorzugsweise 3 bis 8, besonders bevorzugt 4 bis 6 Kohlenstoffatomen aufweisen kann, und
- Oligoether Gruppen der allgemeinen Formel (IV)
-[(CH₂)ₓ-O]_{y}-R¹⁶ (IV),
wobei x und y unabhängig voneinander ausgewählt sind aus ganzen Zahlen zwischen 1 und 250, vorzugsweise 10 und 200, besonders 50 bis 100 und R¹⁶ Wasserstoff oder eine aliphatische, cycloaliphatische, aromatische oder araliphatische Gruppe gemäß den vorherigen für R⁵ bis R¹⁰ getroffenen Definitionen darstellt.

7. Verfahren gemäß irgendeinem der Ansprüche 1 bis 5, **dadurch gekennzeichnet, dass** das Kation [X⁺] wie folgt definiert ist: 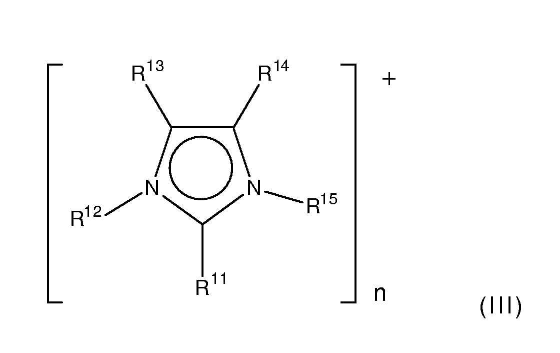 wobei die Reste R¹¹ bis R¹⁵ unabhängig voneinander ausgewählt sind aus der Gruppe bestehend aus
- Wasserstoff;
- aliphatischen geradkettigen oder verzweigten Kohlenwasserstoffresten, mit 1 bis 20, vorzugsweise 2 bis 18, besonders bevorzugt 3 bis 14 Kohlenstoffatomen, die in der Kohlenstoffkette zusätzlich Heteroatome, ausgewählt aus N, S und O, sowie Unsättigungen, in Form von einer, zwei oder mehreren konjugierten oder isolierten Doppelbindungen oder einer isolierten Dreifachbindung aufweisen können, wobei gesättigte aliphatische Gruppen bevorzugt sind,
- cycloaliphatischen Kohlenwasserstoffresten mit 3 bis 20, bevorzugt 5 bis 18, besonders bevorzugt 6 bis 8 Kohlenstoffatomen, wobei die cyclischen Reste Ring-Heteroatome, ausgewählt aus N, S und O, sowie Unsättigungen, in Form von einer, zwei oder mehreren konjugierten oder isolierten Doppelbindungen oder einer isolierten Dreifachbindung aufweisen können,
- aromatischen und araliphatischen Gruppen mit 5 oder 6 bis 22, vorzugsweise 6 bis 14, besonders bevorzugt 10 Kohlenstoffatomen im aromatischen Kern, wobei der aromatische Kern 1 bis 4, vorzugsweise 2 bis 3 Heteroatome, ausgewählt aus N, S und O und lineare oder verzweigte Kohlenwasserstoff-Reste mit 1 bis 10, vorzugsweise 3 bis 8, besonders bevorzugt 4 bis 6 Kohlenstoffatomen aufweisen kann, und
- Oligoethergruppen Gruppen der allgemeinen Formel (IV)
-[(CH₂)ₓ-O]_{y}-R¹⁶ (IV)
sein können, wobei x und y unabhängig voneinander ausgewählt sind aus ganzen Zahlen zwischen 1 und 250, vorzugsweise 10 und 200, besonders 50 bis 100 und R¹⁶ Wasserstoff oder eine aliphatische, cycloaliphatische, aromatische oder araliphatische Gruppe gemäß den vorherigen für R¹¹ bis R¹⁵ getroffenen Definitionen darstellt.

8. Ionische Flüssigkeiten erhältlich nach einem Verfahren wie in irgendeinem der Ansprüche 1 bis 7 definiert.

9. Ionische Flüssigkeit nach Anspruch 8, **dadurch gekennzeichnet, dass** sie einen Gehalt von jeweils < 5 ppm, vorzugsweise < 3 ppm an [Ag⁺] und [I⁻] aufweist.

10. Ionische Flüssigkeit gemäß der allgemeinen Formel (II) 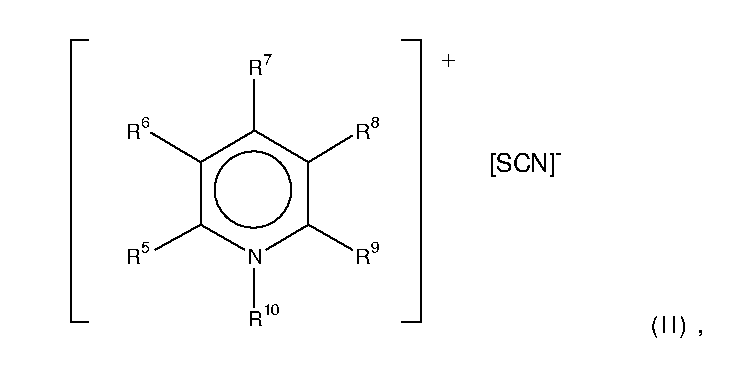 wobei die Reste R⁵ bis R¹⁰ unabhängig voneinander ausgewählt sind aus der Gruppe bestehend aus
- aliphatischen geradkettigen oder verzweigten Kohlenwasserstoffresten, mit 1 bis 20, vorzugsweise 2 bis 18, besonders bevorzugt 3 bis 14 Kohlenstoffatomen, die in der Kohlenstoffkette zusätzlich Heteroatome, ausgewählt aus N, S und O, sowie Unsättigungen, in Form von einer, zwei oder mehreren konjugierten oder isolierten Doppelbindungen oder einer isolierten Dreifachbindung aufweisen können, wobei gesättigte aliphatische Gruppen bevorzugt sind,
- cycloaliphatischen Kohlenwasserstoffresten mit 3 bis 20, bevorzugt 5 bis 18, besonders bevorzugt 6 bis 8 Kohlenstoffatomen, wobei die cyclischen Reste Ring-Heteroatome, ausgewählt aus N, S und O, sowie Unsättigungen, in Form von einer oder mehreren konjugierten oder isolierten Doppelbindungen oder einer isolierten Dreifachbindung aufweisen können,
- aromatischen und araliphatischen Gruppen mit 5 oder 6 bis 22, vorzugsweise 6 bis 14, besonders bevorzugt bis 10 Kohlenstoffatomen im aromatischen Kern, wobei der aromatische Kern 1 bis 4, vorzugsweise 2 bis 3 Heteroatome, ausgewählt aus N, S und O, und lineare oder verzweigte Kohlenwasserstoff-Reste mit 1 bis 10, vorzugsweise 3 bis 8, besonders bevorzugt 4 bis 6 Kohlenstoffatomen aufweisen kann, und
- Oligoether Gruppen der allgemeinen Formel (IV)
-[(CH₂)ₓ-O]_{y}-R¹⁶ (IV),
wobei x und y unabhängig voneinander ausgewählt sind aus ganzen Zahlen zwischen 1 und 250, vorzugsweise 10 und 200, besonders 50 bis 100 und R¹⁶ Wasserstoff oder eine aliphatische, cycloaliphatische, aromatische oder araliphatische Gruppe gemäß den vorherigen für R⁵ bis R¹⁰ getroffenen Definitionen darstellt.

11. Ionische Flüssigkeit gemäß der allgemeinen Formel (III) 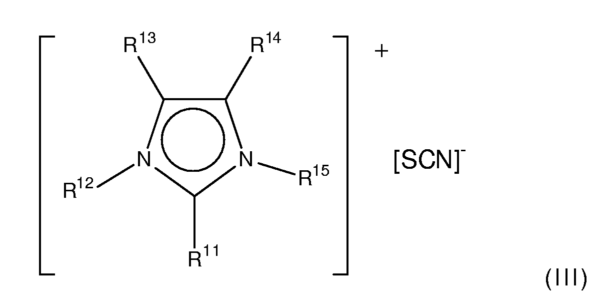 wobei die Reste R¹¹ bis R¹⁵ unabhängig voneinander ausgewählt sind aus der Gruppe bestehend aus
- Wasserstoff;
- aliphatischen geradkettigen oder verzweigten Kohlenwasserstoffresten, mit 1 bis 20, vorzugsweise 2 bis 18, besonders bevorzugt 3 bis 14 Kohlenstoffatomen, die in der Kohlenstoffkette zusätzlich Heteroatome, ausgewählt aus N, S und O, sowie Unsättigungen, in Form von einer, zwei oder mehreren konjugierten oder isolierten Doppelbindungen oder einer isolierten Dreifachbindung aufweisen können, wobei gesättigte aliphatische Gruppen bevorzugt sind,
- cycloaliphatischen Kohlenwasserstoffresten mit 3 bis 20, bevorzugt 5 bis 18, besonders bevorzugt 6 bis 8 Kohlenstoffatomen, wobei die cyclischen Reste Ring-Heteroatome, ausgewählt aus N, S und O, sowie Unsättigungen, in Form von einer, zwei oder mehreren konjugierten oder isolierten Doppelbindungen oder einer isolierten Dreifachbindung aufweisen können,
- aromatischen und araliphatischen Gruppen mit 5 oder 6 bis 22, vorzugsweise 6 bis 14, besonders bevorzugt 10 Kohlenstoffatomen im aromatischen Kern, wobei der aromatische Kern 1 bis 4, vorzugsweise 2 bis 3 Heteroatome, ausgewählt aus N, S und O und lineare oder verzweigte Kohlenwasserstoff-Reste mit 1 bis 10, vorzugsweise 3 bis 8, besonders bevorzugt 4 bis 6 Kohlenstoffatomen aufweisen kann, und
- Oligoethergruppen Gruppen der allgemeinen Formel (IV)
-[(CH₂)ₓ-O]_{y}-R¹⁶ (IV)
sein können, wobei x und y unabhängig voneinander ausgewählt sind aus ganzen Zahlen zwischen 1 und 250, vorzugsweise 10 und 200, besonders 50 bis 100 und R¹⁶ Wasserstoff oder eine aliphatische, cycloaliphatische, aromatische oder araliphatische Gruppe gemäß den vorherigen für R¹¹ bis R¹⁵ getroffenen Definitionen darstellt.

12. Ionische Flüssigkeit, gemäß irgendeinem der Ansprüche 11 oder 12, ausgewählt aus der Gruppe bestehend aus 1,3-Dimethylimidazoliumthiocyanat, 1-Methyl-3-propylimidazoliumthiocyanat, 1-Butyl-3-methylimidazoliumthiocyanat, 1-Hexyl-3-methylimidazoliumthiocyanat, 1-Methyl-3-octylimidazoliumthiocyanat, 1 Decyl-3-methylimidazoliumthiocyanat, 1-Methyl-3-methoxyethylimidazoliumthiocyanat, 1-Methyl-3-ethoxyethylimidazoliumthiocyanat, 1 -Methylpyridiniumthiocyanat, 1-Ethylpyridiniumthiocyanat, 1-Propylpyridiniumthiocyanat, 1-Butyipyridinium-thiocyanat, 1-Hexylpyridiniumthiocyanat, 1-Octylpyridiniumthiocyanat, 1-Decylpyridiniumthiocyanat, 1-Methyoxyethylpyridiniumthiocyanat, 1-Ethoxyethylpyridiniumthiocyanat.

13. Verwendung der ionischen Flüssigkeiten gemäß irgendeinem der Ansprüche 8 bis 12 als Lösungsmittel oder Lösungsmittelzusatz, Phasentransferkatalysator oder Extraktionsmittel in chemischen Reaktionen oder Stofftrennverfahren, als Elektrolyt in einer Batterie oder einem Kondensator oder als Bestandteil einer Farbstoffsolarzelle oder eines Sensors, als oberflächenaktive Substanz oder Weichmacher.
